# EUROPEAN PATENT SPECIFICATION

(11) **EP 2 019 965 B1**
(45) Date of publication and mention of the grant of the patent: **15.04.2015**
(21) Application number: 07761742.1
(22) Date of filing: 02.05.2007
(51) Int. Cl.: G01N 33/53, G01N 33/543, C07K 14/54, C07K 14/58

(54) **DIFFERENTIAL DIAGNOSIS BETWEEN PULMONARY AND CARDIOVASCULAR DISEASE**
DIFFERENTIALDIAGNOSE ZWISCHEN LUNGEN- UND HERZ-KREISLAUF-ERKRANKUNG
DIAGNOSTIC DIFFÉERENTIEL ENTRE MALADIE PULMONAIRE ET LES MALADIES CARDIOVASCULAIRES

(30) Priority: 02.05.2006 US 797285 P
(43) Date of publication of application: 04.02.2009
(73) Proprietor: Critical Care Diagnostics, Inc., New York, New York 10019 (US)
(72) Inventor: SNIDER, James, V., Pleasanton, CA 94588 (US); JACOBSON, Sven, New York, NY 10001 (US)
(74) Representative: Fleck, Barbara
(86) International application number: PCT/US2007/068024
(87) International publication number: WO 2007/131031

(56) References cited:
- US-A1- 2004 048 286
- US-A1- 2004 121 343
- US-A1- 2005 250 156
- WEINBERG E O ET AL: "Identification of serum soluble ST2 receptor as a novel heart failure biomarker" CIRCULATION, LIPPINCOTT WILLIAMS & WILKINS, US, vol. 107, no. 5, 1 February 2003 (2003-02-01), pages 721-726, XP002985431 ISSN: 0009-7322
- SHIMPO MASAHISA ET AL: "Serum levels of the interleukin-1 receptor family member ST2 predict mortality and clinical outcome in acute myocardial infarction" CIRCULATION, vol. 109, no. 18, 11 May 2004 (2004-05-11), pages 2186-2190, XP002552702 ISSN: 0009-7322
- OSHIKAWA KATSUHISA ET AL: "Elevated soluble ST2 protein levels in sera of patients with asthma with an acute exacerbation" AMERICAN JOURNAL OF RESPIRATORY AND CRITICAL CARE MEDICINE, vol. 164, no. 2, 15 July 2001 (2001-07-15), pages 277-281, XP002552703 ISSN: 1073-449X
- CONKLIN B: "B-type Natriuretic Peptide: A New Measurement to Distinguish Cardiac From Pulmonary Causes of Acute Dyspnea" JOURNAL OF EMERGENCY NURSING, C.V. MOSBY, ST. LOUIS, MO, US, vol. 31, no. 1, 1 January 2005 (2005-01-01), pages 73-75, XP004767729 ISSN: 0099-1767
- BAYÉS-GENÍS ANTONI: "[The circulating NTproBNP level, a new biomarker for the diagnosis of heart failure in patients with acute shortness of breath]" REVISTA ESPAÑOLA DE CARDIOLOGÍA OCT 2005, vol. 58, no. 10, October 2005 (2005-10), pages 1142-1144, XP002552704 ISSN: 0300-8932
- PASCUAL FIGAL DOMINGO A ET AL: "[Usefulness of NTproBNP in the emergency management of patients with severe dyspnea and an uncertain heart failure diagnosis]" REVISTA ESPAÑOLA DE CARDIOLOGÍA OCT 2005, vol. 58, no. 10, October 2005 (2005-10), pages 1155-1161, XP002552705 ISSN: 0300-8932
- TUNG ET AL: "Amino-Terminal Pro-Brain Natriuretic Peptide for the Diagnosis of Acute Heart Failure in Patients With Previous Obstructive Airway Disease" ANNALS OF EMERGENCY MEDICINE, LANSING, MI, US, vol. 48, no. 1, 20 February 2006 (2006-02-20), pages 66-74, XP005505827 ISSN: 0196-0644
- SCHMITZ J ET AL: "IL-33, an interleukin-1-like cytokine that signals via the IL-1 receptor-related protein ST2 and induces T helper type 2-associated cytokines" IMMUNITY, CELL PRESS, US, vol. 23, no. 5, 1 November 2005 (2005-11-01), pages 479-490, XP002432892 ISSN: 1074-7613
- TAJIMA SHUNJI ET AL: "The increase in serum soluble ST2 protein upon acute exacerbation of idiopathic pulmonary fibrosis." CHEST, vol. 124, no. 4, October 2003 (2003-10), pages 1206-1214, XP002552706 ISSN: 0012-3692
- JANUZZI JAMES L JR ET AL: "Utility of amino-terminal pro-brain natriuretic peptide testing for prediction of 1-year mortality in patients with dyspnea treated in the emergency department." ARCHIVES OF INTERNAL MEDICINE 13 FEB 2006, vol. 166, no. 3, 13 February 2006 (2006-02-13), pages 315-320, XP008105037 ISSN: 0003-9926
- GEGENHUBER A ET AL: "B-type natriuretic peptide and amino terminal proBNP predict one-year mortality in short of breath patients independently of the baseline diagnosis of acute destabilized heart failure" CLINICA CHIMICA ACTA, ELSEVIER BV, AMSTERDAM, NL, vol. 370, no. 1-2, 6 March 2006 (2006-03-06), pages 174-179, XP025058912 ISSN: 0009-8981 [retrieved on 2006-08-01]
- GOETZE J P ET AL: "B-type natriuretic peptide and its precursor in cardiac venous blood from failing hearts" EUROPEAN JOURNAL OF HEART FAILURE, ELSEVIER, AMSTERDAM, NL, vol. 7, no. 1, 1 January 2005 (2005-01-01), pages 69-74, XP004709133 ISSN: 1388-9842
- MUELLER CHRISTIAN ET AL: "Use of B-type natriuretic peptide in the evaluation and management of acute dyspnea." NEW ENGLAND JOURNAL OF MEDICINE, vol. 350, no. 7, 12 February 2004 (2004-02-12), pages 647-654, XP002552707 ISSN: 0028-4793
- T. Socrates ET AL: "Interleukin family member ST2 and mortality in acute dyspnoea", Journal of Internal Medicine, vol. 268, no. 5, 15 July 2010 (2010-07-15) , pages 493-500, XP055143605, ISSN: 0954-6820, DOI: 10.1111/j.1365-2796.2010.02263.x
- Domingo A. Pascual-Figal ET AL: "Soluble ST2, high-sensitivity troponin T- and N-terminal pro-B-type natriuretic peptide: complementary role for risk stratification in acutely decompensated heart failure", European Journal of Heart Failure, vol. 13, no. 7, 1 July 2011 (2011-07-01), pages 718-725, XP055143607, ISSN: 1388-9842, DOI: 10.1093/eurjhf/hfr047

## Description

### TECHNICAL FIELD

This invention relates to methods for determining the likelihood of the presence of cardiovascular disease (CVD) or pulmonary disease (PD) in a subject using ST2 (which is also known as Interleukin 1 Receptor Like 1 (IL1RL1)) and a diagnostic biomarker, e.g., a natriuretic peptide, e.g., brain natriuretic peptide (BNP)..

### BACKGROUND

Non-specific symptoms such as dyspnea and chest pain are a common problem in the outpatient primary care setting. Establishing a diagnosis can be challenging because the differential diagnosis can include multiple diagnostic categories, including cardiovascular and pulmonary diseases. Underlying disorders can range from relatively benign conditions (e.g., hyperventilation) to more serious and even life-threatening diseases (e.g., pulmonary embolism or heart failure), which are best addressed in an emergency department. Timely assessment, accurate diagnosis, and initiation of appropriate therapy play a critical role in optimizing treatment and patient recovery.

Weinberg et al., (2003) describes a study to test the hypothesis that soluble ST2 levels in the serum of patients with severe chronic heart failure are increased in patients with neurohormonal activation. The results from the study suggested that serum soluble ST2 is a novel biomarker for neurohormonal activation in patients with heart failure and that furthermore, in patients with severe chronic NYHA class III to IV heart failure, the change in ST2 levels is an independent predictor of subsequent mortality or transplantation.

Shimpo et al., (2004) describes a study to test the hypothesis that serum ST2 levels are associated with the risk of death or heart failure in patients presenting with ST-elevation myocardial infarction (STEMI). The results from this study suggested that serum levels of ST2 predict mortality and heart failure in patients with acute myocardial infarction.

US 2004/0121343 describes methods and compositions able to determine the presence or absence of one, preferably a plurality, of diseases including pulmonary embolism and heart failure that exhibit one ore more similar symptoms, that may include dyspnea.

### SUMMARY

The present invention includes an in vitro method of making a differential diagnosis between cardiovascular disease (CVD) and pulmonary disease (PD) in a subject having chest pain or dyspnea, the method comprising:
determining a level of soluble ST2 (Interleukin 1 Receptor Like 1 (IL1RL1)) in a sample from a subject;
determining a level of brain natriuretic peptide (BNP) in the sample; and
comparing the level of soluble ST2 to a reference level of soluble ST2 in a subject who has neither CVD nor PD, has a high likelihood of having PD or CVD or who has CVD and/or PD;
comparing the level of BNP in the sample to a low threshold level of BNP of 100 pg/mL and a high threshold level of BNP of 500 pg/mL; and
diagnosing a subject having chest pain or dyspnea, a soluble ST2 level less than the reference level of soluble ST2, and a BNP level equal to or greater than 100 pg/mL as having CVD;
diagnosing a subject having chest pain or dyspnea, a soluble ST2 level equal to or greater than the reference level of soluble ST2, and a BNP level that is equal to or less than 500 pg/mL as having PD; or
diagnosing a subject having chest pain or dyspnea, a soluble ST2 level equal to or greater than the reference level of soluble ST2, and a BNP level greater than 500 pg/mL as having CVD;
wherein the sample comprises blood, serum, or plasma.

We also describe herein optionally obtaining a biological sample from the subject; determining a level of a first biomarker selected from the group consisting of ST2 and/or IL-33 in such a sample; determining a level of a second biomarker for a cardiovascular disease (CVD) in the sample; and comparing the levels of the first and second biomarkers in the sample to reference levels. The levels of the biomarkers in the sample as compared to the reference levels is correlated with (i.e., is statistically correlated with) the likelihood that the subject has a pulmonary disease or CVD. In some aspects the subject has a non-specific symptom, e.g., dyspnea or chest-pain, that suggests a diagnosis of a pulmonary disease or CVD, and the biomarker levels indicate which of the two diseases, if any, the subject has.

The CVD biomarker is a biomarker that is diagnostic of a cardiovascular condition. TheCVD biomarker may be a natriuretic peptide (NP), e.g., brain natriuretic peptide (BNP), prohormone BNP (proBNP), N-terminal proBNP (NT-proBNP), atrial natriuretic peptide (ANP), proANP, or NT-proANP. In one aspect, the CVD biomarker is BNP.

The biological sample may comprise blood, serum, plasma, urine, or body tissue. In one aspect the sample is a serum sample.

In some aspects of the methods described herein, the reference levels represent levels in a subject who does not have a CVD or a PD. In some aspects the reference levels represent levels in a subject who has a CVD. In some aspects the reference levels represent levels in a subject who has a PD, or both a PD and a CVD.

Determining a level of a biomarker in the sample can include contacting a binding composition to the sample, wherein the binding composition specifically binds to the biomarker, and measuring or determining the specific binding of the binding composition to the sample. Suitable binding compositions include antibodies that bind specifically to a biomarker polypeptide and oligonucleotide probes that bind specifically to a polynucleotide encoding a biomarker.

In some aspects, the subject has dyspnea and the CVD diagnosed by a method described herein is congestive heart failure, coronary artery disease (CAD), arrhythmia, pericarditis, acute myocardial infarction, or anemia.

In some aspects the subject has dyspnea and the pulmonary disease diagnosed by methods described herein is chronic obstructive pulmonary disease (COPD), asthma, pneumonia, pneumothorax, pulmonary embolism, pleural effusion, metastatic disease, pulmonary edema, gastrooesophageal reflux disease with aspiration, or restrictive lung disease.

In some aspects the methods described herein also include determining a level in the sample of one or more other additional biomarkers, e.g., biomarkers selected from the group consisting of troponin, myoglobin, creatine kinase MB (CK-MB), ischemia-modified albumin (IMA), Interleukin-6 (IL-6), C-reactive protein (CRP), creatinine, D-dimers, blood urea nitrogen (BUN), liver function enzymes, albumin, and bacterial endotoxin.

We also describe herein kits for diagnosing pulmonary diseases. The kits include one or more separate antibodies that each specifically bind to a biomarker used in the methods described herein, and/or an oligonucleotide probe that specifically binds to a nucleic acid encoding said biomarkers, and instructions for use in a method described herein.

Also described herein are kits for determining the likelihood of the presence of cardiovascular disease (CVD) or pulmonary disease (PD) in a subject. The kits include (i) one or both of a binding composition that specifically binds to IL-33 and a binding composition that specifically binds to ST2, (ii) a binding composition binds to a biomarker for CVD, and (iii) instructions for use in a method of determining the likelihood of the presence of CVD or PD in a subject described herein. The IL-33 and/or ST2 and the CVD biomarker may comprise polypeptides, and the binding composition includes an antibody or antigen binding fragment thereof that binds specifically to each of the polypeptides. In some aspects, the IL-33 and/or ST2 and the CVD biomarker may comprise a nucleic acid or a nucleic acid probe that binds specifically to each of the nucleic acids.

A "cardiovascular disease," as used herein, refers to a disorder of the heart and blood vessels, and includes disorders of the arteries, veins, arterioles, venules, and capillaries. Cardiovascular diseases diagnosed by a method described herein can include congestive heart failure (HF), coronary artery disease (CAD), arrhythmia, pericarditis, and acute myocardial infarction (MI).

A "pulmonary disease," as used herein, refers to a disorder of the lungs. Pulmonary diseases diagnosed by methods described herein can include chronic obstructive pulmonary disease (COPD), asthma, pneumonia, pneumothorax, pulmonary embolism, pleural effusion, metastatic disease, pulmonary edema, gastrooesophageal reflux disease with aspiration, and/or restrictive lung disease.

"Upregulated," as used herein, refers to increased expression of a gene and/or its encoded polypeptide. "Increased expression" refers to increasing (i.e., to a detectable extent) replication, transcription, and/or translation of a gene, e.g., ST2, since upregulation of any of these processes results in an increase in concentration/amount of the polypeptide encoded by the gene. Conversely, "downregulation," or "decreased expression" as used herein, refers to reduced replication, transcription, and/or translation of the gene and/or its encoded polypeptide. The upregulation or downregulation of gene expression can be directly determined by detecting an increase or decrease, respectively, in the level of mRNA for the gene, or the level of protein expression of the gene-encoded polypeptide, using any suitable means known to the art, such as nucleic acid hybridization or antibody detection methods, respectively, and in comparison to controls. "Expression," as used herein, refers to nucleic acid and/or polypeptide expression.

As used herein, a "subject" is a mammal, e.g., a human or non-human mammal. In general, human nucleic acids and polypeptides, or nucleic acid molecules or polypeptides synthesized or generated to have sequences based on a corresponding human nucleic acid or polypeptide sequence, are preferred for use in diagnosing human subjects.

As used herein, a "sample" includes one or more of blood, serum, plasma, urine, and body tissue. In some aspects, a sample is a serum or blood sample.

Unless otherwise defined, all technical and scientific terms used herein have the same meaning as commonly understood by one of ordinary skill in the art to which this disclosure belongs. Methods and materials are described herein for use in the present disclosure other, suitable methods and materials known in the art can also be used. The materials, methods, and examples are illustrative only and not intended to be limiting. In case of conflict, the present specification, including definitions, will control.

Other features and advantages of the invention will be apparent from the following detailed description and figures, and from the claims.

### DESCRIPTION OF DRAWINGS

FIG. 1 is a table illustrating the effect of COPD history on ST2 concentrations in subjects with/without acute HF.
FIG. 2 is a box graph illustrating levels of ST2 in subjects with COPD/Asthma and/or HF.

### DETAILED DESCRIPTION

Accurate diagnosis of subjects presenting with non-specific symptoms can be difficult, as the symptoms can have very different etiology. For example, subjects with dyspnea (shortness of breath, or uncomfortable breathing) may be suffering from a pulmonary disease or a cardiovascular disease, both, or neither.

### General Methodology

In general, the methods described herein include evaluating levels of a first biomarker (ST2 and/or IL-33) and a second, CVD biomarker (e.g., a natriuretic peptide (NP)) in a biological sample (e.g., a blood, serum, plasma, urine, or body tissue sample) from a subject, e.g., a mammal, e.g., a human. These levels can provide diagnostic information, e.g., indicating whether the subject has a pulmonary disease (PD) or a cardiovascular disease (CVD), as described herein. In some aspects, the second biomarker is brain natriuretic peptide (BNP.

In one example, diagnosis of CVD versus PD can be made by referring to Table 1.

**Table 1: Diagnosis of CVD vs PD**

| | **Low ST2** | **High ST2** |
|---|---|---|
| | **(e.g., < 0.20 ng/mL serum)** | **(e.g., ≥ 0.20 ng/mL serum)** |
| **Low BNP** **(< 100 pg/mL)** | **low probability of either CVD or PD** | **probable PD** |
| **Moderate BNP** **(100-500 pg/ml)** | **possible CVD** | **possible PD** |
| **High BNP** **(> 500 pg/ml)** | **probable CVD** | **highly probable CVD** |

Thus, for a subject who has both low levels of a CVD biomarker, e.g., BNP, and low levels of ST2, there is a low probability of the presence of either CVD or PD. Low levels of the CVD biomarker and high levels of ST2 indicate a greater likelihood of PD than CVD.

For a subject who has moderate levels of BNP, low levels of ST2 indicate that the presence of CVD is possible (and more likely than PD), while high levels of ST2 indicate that the presence of PD is possible (and more likely than CVD).

Finally, for a subject who has high levels of a CVD biomarker, low levels of ST2 indicate that CVD is probable (and more likely than PD), and high levels of ST2 indicate that CVD is highly probable (and more likely than PD).

Using the methods described herein, diagnoses can be ruled in or ruled out for a given subject, allowing a care giver to focus diagnostic efforts, and thus therapeutic efforts, appropriately. Although it is possible that both etiologies may exist simultaneously in a subject, at any given point in time (and particularly in an acute care situation such as presentation with dyspnea) it is likely one etiology would be more "dominant" than the other, and thus would be the primary target for treatment.

The levels of the biomarkers may be determined once, e.g., at presentation. In some aspects, the levels of the biomarkers are determined at any one or more of 1, 2, 3, 4, 5, 6, 7, 8, 12, 18, and/or 24 hours, and/or at 1-7 days or longer, after the onset of symptoms.

Where the levels of the biomarkers are determined more than once, the highest level or an average can be used, or the change in levels can be determined and used. Levels of the biomarkers can also be determined multiple times to evaluate a subject's response to a treatment. For example, levels, e.g., of IL-33 and/or ST2, that are taken after administration of a treatment, e.g., one or more doses or rounds of a treatment, can be compared to levels taken before the treatment was initiated, e.g., baseline levels. The change in levels would indicate whether the treatment was effective; e.g., a reduction in levels would indicate that the treatment was effective.

Evaluating levels of the biomarker in a subject typically includes obtaining a biological sample, e.g., serum or blood, from the subject. Levels of the biomarkers in the sample can be determined by measuring levels of biomarker polypeptides in the sample, using methods known in the art and/or described herein, e.g., immunoassays such as enzyme-linked immunosorbent assays (ELISA). Alternatively, levels of mRNA encoding the biomarkers can be measured, again using methods known in the art and/or described herein, e.g., by quantitative PCR or Northern blotting analysis.

For example, a method as described herein, e.g., for differential diagnosis of pulmonary disease, can include contacting a sample from a subject, e.g., a sample including blood, serum, plasma, urine, or body tissue from the subject, with a binding composition (e.g., an antibody or oligonucleotide probe) that specifically binds to a polypeptide or nucleic acid of the biomarkers as described herein. The methods can also include contacting a sample from a control subject, normal subject, or normal tissue or fluid from the test subject, with the binding composition, e.g., to provide a reference or control. Moreover, the method can additionally include comparing the specific binding of the composition to the test subject with the specific binding of the composition to the normal subject, control subject, or normal tissue or fluid from the test subject. Expression or activity of biomarkers in a test sample or test subject can also be compared with that in a control sample or control subject. A control sample can include, e.g., a sample from a non-affected subject, or a subject who has a known condition, e.g., a pulmonary disease or a cardiovascular disease. Expression or activity from a control subject or control sample can be provided as a predetermined value, e.g., acquired from a statistically appropriate group of control subjects.

An antibody that "binds specifically to" an antigen, binds preferentially to the antigen in a sample containing other proteins. The term "antibody" as used herein refers to an immunoglobulin molecule or immunologically active portion thereof, i.e., an antigen-binding portion. Examples of immunologically active portions of immunoglobulin molecules include F(ab) and F(ab')₂ fragments which can be generated by treating the antibody with an enzyme such as pepsin. The antibody can be polyclonal, monoclonal, recombinant, e.g., a chimeric or humanized, fully human, non-human, e.g., murine, monospecific, or single chain antibody. In some aspects it has effector function and can fix complement.

An "oligonucleotide probe" (also referred to simply as a "probe") is a nucleic acid that is at least 10, and less than 200 (typically less than about 100 or 50) base pairs in length. A probe that "binds specifically to" a target nucleic acid hybridizes to the target under high stringency conditions. As used herein, the term "hybridizes under high stringency conditions" describes conditions for hybridization and washing. As used herein, high stringency conditions are 0.5 M sodium phosphate, 7% SDS at 65°C, followed by one or more washes at 0.2X SSC, 1% SDS at 65°C. Methods for performing nucleic acid hybridization assays are known to those skilled in the art and can be found in Current Protocols in Molecular Biology, John Wiley & Sons, N.Y. (1989), 6.3.1-6.3.6.

Detection can be facilitated by coupling (i.e., physically linking) the antibody or probe to a detectable substance (i.e., antibody labeling). Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride, quantum dots, or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

Diagnostic assays can be used with biological matrices such as live cells, cell extracts, cell lysates, fixed cells, cell cultures, bodily fluids, or forensic samples. Conjugated antibodies useful for diagnostic or kit purposes, include antibodies coupled to dyes, isotopes, enzymes, and metals, see, e.g., Le Doussal et al., New Engl. J. Med. 146:169-175 (1991); Gibellini et al., J. Immunol. 160:3891-3898 (1998); Hsing and Bishop, New Engl. J. Med. 162:2804-2811 (1999); Everts et al., New Engl. J. Med. 168:883-889 (2002). Various assay formats exist, such as radioimmunoassays (RIA), ELISA, and lab on a chip (U.S. Pat. Nos. 6,176,962 and 6,517,234).

Known techniques in biochemistry and molecular biology can be used in the methods described herein (see, e.g., Maniatis et al., Molecular Cloning, A Laboratory Manual, Cold Spring Harbor Laboratory Press, Cold Spring Harbor, N.Y. (1982); Sambrook and Russell, Molecular Cloning, 3rd ed., Cold Spring Harbor Laboratory Press, Cold Spring Harbor, N.Y. (2001); Wu, Recombinant DNA, Vol. 217, Academic Press, San Diego, Calif (1993); and Ausbel et al., Current Protocols in Molecular Biology, Vols. 1-4, John Wiley and Sons, Inc. New York, N.Y. (2001)).

Once a level of a biomarker has been determined, the level can be compared to a reference level. In some aspects, the reference level will represent a threshold level, above which the subject can be diagnosed with CVD or PD disease. The reference level chosen may depend on the methodology used to measure the levels of the biomarkers. In some aspects, the reference level is a range of levels.

We also describe herein that both levels of ST2 and IL-33 may be determined, and the information from the comparison of both biomarkers with their respective reference levels provides cumulative information regarding the presence of pulmonary disease in the subject, and/or the presence of a severe disease in the subject. In some aspects, the ratio of ST2 to IL-33 may be determined, and the ratio compared to a reference ratio that represents a threshold ratio above which the subject has CVD or PD, e.g., as shown in Table 1.

### ST2/Interleukin 1 Receptor-Like 1 (IL1RL1)

The ST2 gene is a member of the interleukin-1 receptor family, whose protein product exists both as a trans-membrane form, as well as a soluble receptor that is detectable in serum (Kieser et al., FEBS Lett. 372(2-3):189-93 (1995); Kumar et al., J. Biol. Chem. 270(46):27905-13 (1995); Yanagisawa et al., FEBS Lett. 302(1):51-3 (1992); Kuroiwa et al., Hybridoma 19(2):151-9 (2000)). ST2 was recently described to be markedly up-regulated in an experimental model of heart failure (Weinberg et al., Circulation 106(23):2961-6 (2002)), and preliminary results suggest that ST2 concentrations may be elevated in those with chronic severe HF (Weinberg et al., Circulation 107(5):721-6 (2003)) as well as in those with acute myocardial infarction (MI) (Shimpo et al., Circulation 109(18):2186-90 (2004)).

The transmembrane form of ST2 is thought to play a role in modulating responses of T helper type 2 cells (Lohning et al., Proc. Natl. Acad. Sci. U. S. A. 95(12):6930-5 (1998); Schmitz et al., Immunity 23(5):479-90 (2005)), and may play a role in development of tolerance in states of severe or chronic inflammation (Brint et al., Nat. Immunol. 5(4):373-9 (2004)), while the soluble form of ST2 is up-regulated in growth stimulated fibroblasts (Yanagisawa et al., 1992, supra). Experimental data suggest that the ST2 gene is markedly up-regulated in states of myocyte stretch (Weinberg et al., 2002, supra) in a manner analogous to the induction of the BNP gene (Bruneau et al., Cardiovasc. Res. 28(10):1519-25 (1994)).

Tominaga, FEBS Lett. 258:301-304 (1989), isolated murine genes that were specifically expressed by growth stimulation in BALB/c-3T3 cells; they termed one of these genes St2 (for Growth Stimulation-Expressed Gene 2). The St2 gene encodes two protein products: ST2 (IL1RL1), which is a soluble secreted form; and ST2L, a transmembrane receptor form that is very similar to the interleukin-1 receptors. The HUGO Nomenclature Committee designated the human homolog, the cloning of which was described in Tominaga et al., Biochim. Biophys. Acta. 1171:215-218 (1992), as Interleukin 1 Receptor-Like 1 (IL1RL1). The two terms (ST2 and IL1RL1) are used interchangeably herein.

The mRNA sequence of the shorter, soluble isoform of human ST2 can be found at GenBank Acc. No. NM_003856.2, and the polypeptide sequence is at GenBank Acc. No. NP_003847.2; the mRNA sequence for the longer form of human ST2 is at GenBank Acc. No. NM_016232.4; the polypeptide sequence is at GenBank Acc. No. NP_057316.3. Additional information is available in the public databases at GeneID: 9173, MIM ID # 601203, and UniGene No. Hs.66. In general, in the methods described herein, the soluble form of ST2 polypeptide is measured.

Methods for detecting and measuring ST2 are known in the art, e.g., as described in U.S. Pat. Pub. Nos. 2003/0124624,2004/0048286 and 2005/0130136. Kits for measuring ST2 polypeptide are also commercially available, e.g., the ST2 ELISA Kit manufactured by Medical & Biological Laboratories Co., Ltd. (MBL International Corp., Woburn, MA), no. 7638. In addition, devices for measuring ST2 and other biomarkers are described in U.S. Pat. Pub. No. 2005/0250156.

In one aspect, the level of ST2 is determined once, e.g., at presentation. In some aspects, the level of ST2 is determined at one or more of 2, 4, 6, 8, 12, 18, and/or 24 hours, and/or 1-7 days after the onset of symptoms.

We also describe herein that the level of ST2 may be determined more than once; in that case, the higher measurement can be used. Where the level of ST2 is determined more that once, the highest level can be used, or the change in levels can be determined and used. Levels of ST2 can also be determined multiple times to evaluate a subject's response to a treatment. For example, a level of ST2 taken after administration of a treatment, e.g., one or more doses or rounds of a treatment, can be compared to levels of ST2 before the treatment was initiated, e.g., a baseline level. The change in ST2 levels would indicate whether the treatment was effective; e.g., a reduction in ST2 levels would indicate that the treatment was effective.

The methods may also include determining the identity of the nucleotide sequence at RefSNP ID: rs1041973.

### Interleukin-33 (IL-33)

IL-33 was recently identified as the ligand for ST2, and the presence of increased levels of IL-33 in various inflammatory disorders has been described (see Schmitz et al., Immunity 23(5):479-90 (2005); U.S. Pat. Pub. No. 2005/0203046). In the methods described herein, ST2 can be measured in addition to IL-33. The ratio of ST2 to IL-33 can also be determined, as can ratios of bound complexes to bound and/. IL-33 protein is expressed as an inactive molecule, pre-IL-33, that is activated after cleavage by Caspase I resulting in the active IL-33 peptide as well as the cleavage peptide product, pro-IL-33. Therefore, the methods described herein can include measuring one, two, or all three of mature IL-33, pre-IL-33, and/or pro-IL-33, all of which are included in the term "IL-33."

The nucleic acid sequence of IL-33 can be found at GenBank Acc. No. NM_033439.2, and the polypeptide sequence is at GenBank Acc. No. NP_254274.1. Additional information is available in the public databases at GeneID: 90865, MIM ID # *608678, and UniGene No. Hs.348390. IL-33 is also known as Chromosome 9 Open Reading Frame 26 (C9ORF26); Nuclear Factor from High Endothelial Venules (NFHEV); and Interleukin 33. See also Baekkevold et al., Am. J. Path. 163: 69-79 (2003).

Methods for measuring levels of IL-33 polypeptide and nucleic acid are known in the art, see, e.g., Schmitz et al., Immunity 23(5):479-90 (2005); U.S. Pat. Pub. No. 2005/0203046.

### CVD Biomarkers

The methods described herein include measuring levels of CVD biomarkers in addition to IL1RL1 (ST2) and/or IL-33. Suitable biomarkers for CVD include troponin, NT-proBNP, BNP NT-proANP, and ANP.

In some aspects the CVD diagnostic biomarker is B-type natriuretic peptide (BNP), a marker of hemodynamic stress characteristic of heart failure. Levels of BNP can be determined, e.g., in whole blood or serum, using standard methodology. For example, a number of assay kits are commercially available, e.g., the Triage BNP Test (Biosite, Inc., San Diego, CA), a point-of-care assay that whole blood or plasma and produces results in about 15 minutes; a chemiluminescent sandwich immunoassay (Bayer HealthCare Diagnostics, Tarrytown, NY) for BNP that is run on the ADVIA Centaur and ACS:180 platforms; a microparticle-based immunoassay (Abbott Laboratories, Abbott Park, IL) for BNP that is run on the AxSYM platform; and a chemiluminescent immuno-enzymatic assay (Biosite, Inc., San Diego, CA) for BNP that is run on the following Beckman Coulter platforms: Access, Access 2, Synchron LXI and the UniCel DXI. An electrochemiluminescent assay (Roche Diagnostics, Indianapolis, IN) available for measuring NT-proBNP.

The reference ranges for BNP and NTproBNP vary depending on a number of factors. The following ranges are for use where BNP levels are measured using an ELISA-type method, and one of skill in the art will be able to determine what levels obtained using other methods are equivalent. If the BNP level is >500 pg/mL, then HF is highly likely. Levels of BNP of 100-500 pg/mL are often described as a "grey zone," in which diagnosis is less certain. In lean subjects, if the BNP is <100 pg/mL, then HF is unlikely, however, obesity influences the expression of BNP in chronic HF (Mehra et al., J Am Coll Cardiol. 43(9):1590-1595 (2004)), so levels of < 100 pg/mL do not rule out heart failure in obese subjects (Silver et al., Cong. Heart Fail. 10(5 suppl. 3):1-30 (2004)).

### Other Biomarkers

We also describe herein methods for measuring levels of other biomarkers, e.g., one or more of: troponin, creatine kinase MB (CK-MB), Myoglobin (Myo), ischemia-modified albumin (IMA), Interleukin-6 (IL-6), C-reactive protein (CRP), creatinine, D-dimers, blood urea nitrogen (BUN), liver function enzymes, albumin, and/or bacterial endotoxin. Methods for measuring these biomarkers are known in the art, see, e.g., U.S. Pat. Pub. Nos. 2004/0048286 and 2005/0130136 to Lee et al.; Dhalla et al., Mol. Cell Biochem. 87:85-92 (1989); Moe et al., Am. Heart J. 139:587-95 (2000).

### Kits

Also described herein are kits that include a reagent comprising a binding composition for the detection of one or more of the IL-33 or ST2 polypeptide(s) or nucleic acid, e.g., an anti-IL-33 or ST2 antibody (i.e., an antibody or antigen binding fragment thereof that binds specifically to IL-33 or ST2), or a nucleic acid probe complementary to all or part of the IL-33 or ST2 nucleic acid), as well as a reagent comprising a binding composition for the detection of one or more of a CVD biomarker, e.g., a CVD biomarker polypeptide or a nucleic acid encoding a CVD biomarker, and instructions for use in a method described herein. A control can also be included, e.g., an epitope of IL-33 or ST2, and of the CVD biomarker.

Kits are generally comprised of the following major elements: packaging, reagents comprising binding compositions as described above, optionally a control, and instructions. Packaging may be a box-like structure for holding a vial (or number of vials) containing said binding compositions, a vial (or number of vials) containing a control, and instructions for use in a method described herein. Individuals skilled in the art can readily modify the packaging to suit individual needs.

As one example, the kit may contain an antibody or antigen binding fragment thereof that binds specifically to ST2 (or IL-33), and an antibody or antigen binding fragment thereof that binds specifically to a CVD biomarker, e.g., BNP, proBNP, NT-proBNP, ANP, proANP, or NT-proANP.

We also describe that other methods of detection can be used, e.g., colorimetric assays, radioimmunoassays, or chemiluminescent assays. Sandwich assays can be used as well, e.g., using two monoclonal antibodies, one labelled with iodine 125 and the other adsorbed onto beads, e.g., as used in the IRMA-BNP2 kit from CISBIO International (France) and the ShionoRIA BNP or ANP kits (SHIONOGI USA Inc.).

For example, the kit can be designed for use in a assay is a chemiluminescent microparticle immunoassay (CMIA), such as the ARCHITECT assays from Abbot Diagnostics (Abbott Park, IL), and thus can contain paramagnetic microparticles coated with anti-BNP antibodies, and paramagnetic microparticles coated with anti-ST2 antibodies. These microparticles are contacted with a sample, and the BNP and ST2 present in the sample bind to the coated microparticles. Optionally, the sample can be split into at least two aliquots, and each type of microparticle can be contacted with a separate aliquot. After washing, anti-BNP and anti-ST2 acridinium-labeled conjugate can be added to create a reaction mixture in the second step. Following another wash cycle pre-trigger and trigger solutions are added to the reaction mixture. The resulting chemiluminescent reaction is measured, e.g., using the ARCHITECT i System optics (Abbot Diagnostics, Abbott Park, Illinois). A direct relationship exists between the amount of BNP or ST2 in the sample and the chemiluminescence detected.

### EXAMPLES

### Example 1: Detection and Measurement of ST2 in the Serum

This example uses the ST2 ELISA Kit manufactured by Medical & Biological Laboratories Co., Ltd. (MBL International Corp., Woburn, MA), no. 7638. This kit is a sandwich ELISA assay utilizing monoclonal antibodies for both capture and detection. This procedure is intended to analyze a full plate of samples assayed in replicates at a 1:3 dilution factor and closely follows the manufacturers' protocol. Kits should be stored at 4°C until use. The procedure described in this example is optimized for human serum or plasma collected in citrate or EDTA anticoagulant tubes. Plasma collected in heparin anticoagulant tubes should not be used in this assay as heparin binds ST2 and inhibits measurement by this ELISA protocol. Plasma or serum samples may be used fresh or stored frozen. This assay is not adversely affected by up to 3 freeze and thaw cycles of plasma samples.

Reagents should be prepared fresh from a new kit immediately before performing the assays. Allow the kit to equilibrate to room temperature prior to use. Reagents not explicitly discussed below are provided by the manufacturer ready to use.
1. Wash solution - wash solution is provided by the manufacturer as a 10X concentrate solution. To make 1 liter of wash solution dilute 100 ml of the 10X concentrate provided with 900 ml of distilled water.
2. Detector solution - the detector solution is prepared by diluting the detector concentrate 1:101 with the detector diluent. For a full 96 well plate of samples 10 ml of detector solution is required. To prepare 10 ml of detector solution use a pipette to transfer 10 ml of the blue colored detector diluent to a 15 ml orange top polypropylene tube. Ad 100 µl of the detector concentrate to this volume of detector diluent.
   a. NOTE: this reagent should be prepared during the first assay incubation step.
3. Calibrator stock - reconstitute the calibrator protein by dissolving the lyophilized protein in the amount of distilled water defined by the manufacturer for this manufacturing lot to yield a stock solution of 8 ng/ml. This volume specification is included in the product insert.

### Preparation of standards and samples:

- All of the following should be prepared in labeled 1.5 ml polypropylene tubes to be transferred to the assay plate with the P200 pipetter.

### Standards:

The standard curve is prepared by making 2 fold serial dilutions of the 8 ng/ml stock solution.
1. Using a P1000 pipette transfer 250 µl of Assay Diluent to 8 1.5 ml polypropylene tubes labeled S1-S8
2. Using the same P1000 pipette transfer 250 µl of the 8 ng/ml Calibrator stock solution to tube S1. This tube is now 4 ng/ml calibrator protein.
   a. Mix thoroughly by gently pipetting 3 times being careful not to create bubbles.
3. Using the same P1000 pipette and a fresh tip for each of the following transfer 250 µl of the reagent in tube S1 to tube S2, repeat the mixing.
4. Repeat step 3 for S2 to S3, S3 to S4, S4 to S5, S5 to S6 and S6 to S7. S8 will be the reagent blank so do not transfer the calibrant protein to this well.
   a. Tubes S1-S6 and S8 will now have 250 µl of reagent and tube S7 will have 450 µl

### Samples:

The plate is set up so that each sample is analyzed as a 1:3 dilution in duplicate.
   1. Label a 1.5 ml polypropylene tube for each sample.
   2. Using the P200 pipette transfer 160 µl of Assay Diluent to each tube.
   3. Using a P200 pipette transfer 80 µl of serum or plasma from sample 1 to tube 1. Mix carefully by pipetting 3 times without making bubbles.
   4. Continue transferring samples to the sample tubes by repeating step 2 for each sample.

### Procedure:

1. Use the P200 pipette transfer the standards and diluted serum samples quickly to the 96 well assay plate. An exemplary layout is shown below in Table 2.
   a. Set the P200 pipette for 100 µl
   b. Transfer 100 µl of the standard curve dilutions to each of columns 1 & 2 in the assay plate
   c. Transfer 100 µl of each of the serum samples to the assay plate in exactly the same positions as shown in the plate map below.
2. Cover the assay plate with the provided shield and incubate at room temperature for 60 minutes.
3. Using the plate autowasher wash the plate 4 times.
4. Detector: using the 8 channel multichannel pipette transfer 100 µl of the detector solution to each well and incubate at room temperature for 60 minutes.
   a. NOTE: this reagent was to be prepared during the first incubation step.
   b. NOTE: use a disposable reagent vessel for this reagent addition.
      ALWAYS use a fresh disposable reagent vessel for each reagent. It is not necessary to change pipette tips during this step.
5. Wash the plate as in step 3
6. Substrate: using the 8 channel multichannel pipette transfer 100 µl of the Substrate to each well and incubate at room temperature for 30 minutes.
   a. The Substrate reagent is provided ready to use by the manufacturer.
7. Stop: at the completion of the Substrate incubation using the 8 channel multichannel pipette transfer 100 µl of the Stop solution to each well.
   a. The Stop Solution reagent is provided ready to use by the manufacturer.
8. Read the plate at 450 nm with background correction at 620 nm.
   a. The plate should be read within 30 minutes after stopping the reaction.
9. Enter the absorbance readings in the provided spreadsheet for analysis.

**Table 2: Map of Exemplary 96 Well Assay Plate**

| | 1 | 2 | 3 | 4 | 5 | 6 | 7 | 8 | 9 | 10 | 11 | 12 |
|---|---|---|---|---|---|---|---|---|---|---|---|---|
| A | 4.0 | | 1 | 1 | 9 | 9 | 17 | 17 | 25 | 25 | 33 | 33 |
| B | 2.0 | | 2 | 2 | 10 | 10 | 18 | 18 | 26 | 26 | 34 | 34 |
| C | 1.0 | | 3 | 3 | 11 | 11 | 19 | 19 | 27 | 27 | 35 | 35 |
| D | 0.5 | | 4 | 4 | 12 | 12 | 20 | 20 | 28 | 28 | 36 | 36 |
| E | 0.25 | | 5 | 5 | 13 | 13 | 21 | 21 | 29 | 29 | 37 | 37 |
| F | 0.125 | | 6 | 6 | 14 | 14 | 22 | 22 | 30 | 30 | 38 | 38 |
| G | 0.0625 | | 7 | 7 | 15 | 15 | 23 | 23 | 31 | 31 | 39 | 39 |
| H | 0.0 | | 8 | 8 | 16 | 16 | 24 | 24 | 32 | 32 | 40 | 40 |

### Example 2: Detection and Measurement of IL-33 in the Serum

A blood sample is collected from a subject, and serum is prepared from the sample using standard methods. A labeled monoclonal antibody to IL-33 (e.g., as described U.S. Pat. App. Pub. No. 2005/020304) is added to the sample and incubated for a sufficient amount of time for binding to occur. The antibody/IL-33 complexes are then detected using standard methods, and the amount of IL-33 present is quantified. Levels of IL-33 are expected to correlate with disease in a manner similar to that of ST2, as described herein.

### Example 3: COPD and ST2 Concentrations

The effect of COPD history on ST2 concentrations in subjects with/without acute HF was evaluated in subjects from the PRIDE study.

600 breathless subjects were enrolled in the PRIDE study, to analyze the utility of NT-proBNP for diagnosis and prognosis of acute heart failure (HF). At enrollment, a blinded sample of blood was obtained, processed and frozen at -80°C. For the purposes of ST2 analysis, an aliquot of citrated blood was thawed (second freeze-thaw cycle) and analyzed for concentration of ST2 protein as described in Example 1.

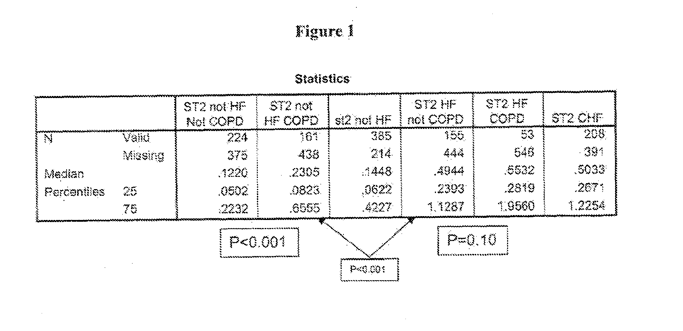

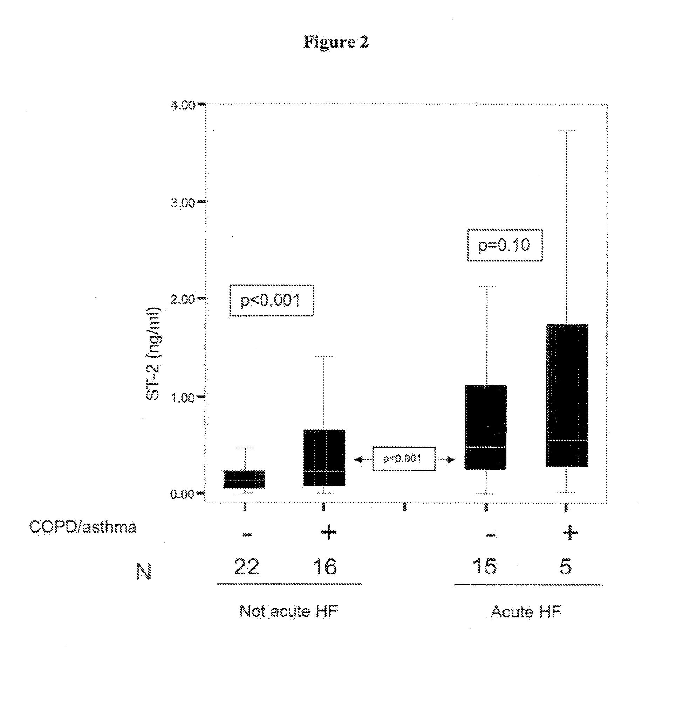

The results are shown in Figures 1-2, and demonstrate that elevated ST2 levels, e.g., above 0.2 ng/ml of serum when determined as described in Example 1, can be used to predict the likelihood of the presence of pulmonary disease, e.g., in subjects without acute decompensated heart failure, e.g., with low or moderate BNP levels.

### Example 4: Elevated ST2 Concentrations in Patients Without Heart Failure

ST2 concentrations were determined as described in Example 1, above, in a population of 350 patients who presented to the ED with chest pain. Serum samples were obtained and ST2 measurements made at baseline, and 90 and 180 minutes later for most patients. Also for most patients, the baseline sample was collected within 2 hours of onset of symptoms.

17 patients had final diagnosis of MI, and 5 of these had ST2 ≥ 0.23 (0.25 - 0.65). Two of these patients were troponin negative. 11 patients had very high ST2 levels (0.97 - 9.22), but none of these patients had confirmed final diagnosis of MI and were all troponin negative, though all had severe diseases, including COPD, lymphoma, sepsis, alcohol abuse, and pulmonary embolism. The ST2 levels and diagnoses for these 11 patients are shown in Table 3; ST2 1 is the baseline level, ST2 2 is 90 minutes later, and ST2 3 is at 180 minutes.

**Table 3: Non-MI Patients with High ST2 Levels**

| **Patient** | **ST2 1** | **ST2 2** | **ST2 3** | **Final Diagnosis** |
|---|---|---|---|---|
| | **(baseline)** | **(90 mins)** | **(180 Mins)** | |
| | **(ng/ml)** | **(ng/ml)** | **(ng/ml)** | |
| 811 | 1.43 | 1.62 | 1.63 | COPD with heart failure following coronary artery bypass graft surgery and pulmonary hypertension |
| 847 | 2.37 | 4.44 | 3.53 | Pulmonary embolism |
| 873 | 2.36 | 2.42 | 2.74 | Reactive airway disease (RAD) |
| 898 | 1.32 | 1.24 | 1.66 | History of heart failure following coronary artery bypass surgery |
| 920 | 6.03 | 9.22 | | Bacteremia sepsis |
| 928 | 3.80 | 4.69 | 3.99 | Hypertension and alcohol abuse |
| 952 | 6.76 | | | Alcohol abuse, gastritis and pulmonary hypertension |
| 953 | 3.77 | | | History of heart failure following coronary artery bypass surgery |
| 1055 | 1.42 | 1.28 | 1.13 | Upper respiratory infection (URI) |
| 1213 | 0.97 | 1.19 | 1.07 | Pulmonary embolism and pericarditis |
| 1245 | 4.11 | 6.46 | | and hypertension |
| 1280 | 1.30 | 1.33 | | COPD |

These results demonstrate that the presence of elevated ST2 (e.g., above 0.2 ng/ml) in patients with chest pain who are troponin negative is associated with a high probability of pulmonary disease.

## Claims

1. An in vitro method of making a differential diagnosis between cardiovascular disease (CVD) and pulmonary disease (PD) in a subject having chest pain or dyspnea, the method comprising:
determining a level of soluble ST2 (Interleukin 1 Receptor Like 1 (IL1RL1)) in a sample from a subject;
determining a level of brain natriuretic peptide (BNP) in the sample; and
comparing the level of soluble ST2 to a reference level of soluble ST2 in a subject who has neither CVD nor PD, has a high likelihood of having PD or CVD, or has CVD and/or PD;
comparing the level of BNP in the sample to a low threshold level of BNP of 100 pg/mL and a high threshold level of BNP of 500 pg/mL; and
diagnosing a subject having chest pain or dyspnea, a soluble ST2 level less than the reference level of soluble ST2, and a BNP level equal to or greater than 100 pg/mL as having CVD;
diagnosing a subject having chest pain or dyspnea, a soluble ST2 level equal to or greater than the reference level of soluble ST2, and a BNP level that is equal to or less than 500 pg/mL as having PD; or
diagnosing a subject having chest pain or dyspnea, a soluble ST2 level equal to or greater than the reference level of soluble ST2, and a BNP level greater than 500 pg/mL as having CVD;
wherein the sample comprises blood, serum, or plasma.

2. The method of claim 1, wherein determining a level of soluble ST2 or the level of BNP in the sample comprises contacting a binding composition to the sample, wherein the binding composition specifically binds to soluble ST2 or BNP, and measuring or determining the specific binding of the binding composition to the sample.

3. The method of claim 2, wherein the binding composition comprises an antibody that binds specifically to soluble ST2 or BNP.

4. The method of claim 1, wherein the CVD is acute coronary syndrome (ACS), myocardial infarction, heart failure, angina, cardiac hypertrophy, myocarditis, pericarditis, endocarditis, or stroke.

5. The method of claim 1, wherein the PD is chronic obstructive pulmonary disease (COPD), asthma, pneumonia, pneumothorax, pleural effusion, metastatic disease, pulmonary edema, gastroesophageal reflux disease with aspiration, pulmonary embolism, or restrictive lung disease.

6. The method of claim 1 or 2, further comprising determining a level in the sample of one or more other biomarkers.

7. The method of claim 6, wherein the one or more other biomarkers are selected from the group consisting of: creatine kinase MB (CK-MB), C-reactive protein (CRP), creatinine, and D-dimers.

## Patentansprüche

1. In vitro-Verfahren zur Differentialdiagnose zwischen Herz-Gefäß-Krankheit (CVD) und Lungenkrankheit (PD) bei einem Probanden mit Brustschmerzen oder Dyspnoe, umfassend:
Ermitteln eines löslichen ST2-Spiegels (Interleukin 1 Receptor Like 1 (IL1RL1)) in einer einem Probanden entnommenen Probe;
Ermitteln eines BNP-Spiegels (Brain Natriuretic Peptide) in der Probe und
Vergleichen des lösliche ST2-Spiegel mit einem Referenzspiegel an löslichem ST2 in einem Probanden, der weder CVD noch PD hat, mit hoher Wahrscheinlichkeit PD oder CVD hat oder tatsächlich CVD und/oder PD hat;
Vergleichen des BNP-Spiegels in der Probe mit einem niedrigen Schwellenwert von 100 pg/mL für BNP und einem hohen BNP-Schwellenwert von 500 pg/mL und
Feststellen von CVD bei einem Probanden mit Brustschmerzen oder Dyspnoe, einem den Referenzspiegel an löslichem ST2 unterschreitenden löslichen ST2-Spiegel und einem BNP-Wert, der größer oder gleich 100 pg/mL ist;
Feststellen von PD bei einem Probanden mit Brustschmerzen oder Dyspnoe, einem ST2-Spiegel, der größer oder gleich dem Referenzspiegel an löslichem ST2 ist, und einem BNP-Wert, der größer oder gleich 500 pg/mL ist; oder
Feststellen von CVD bei einem Probanden mit Brustschmerzen oder Dyspnoe, einem löslichen ST2-Spiegel, der größer oder gleich dem Referenzspiegel an löslichem ST2 ist, und einem BNP-Wert, der größer oder gleich 500 pg/mL ist;
wobei die Probe Blut, Serum oder Plasma umfasst.

2. Verfahren nach Anspruch 1, wobei das Ermitteln eines löslichen ST2-Spiegels oder des BNP-Spiegels in der Probe das Kontaktieren einer Bindungszusammensetzung mit der Probe, wobei die Bindungszusammensetzung spezifisch an lösliches ST2 oder BNP bindet, und Messen oder Bestimmen der spezifischen Bindung der Bindungszusammensetzung an die Probe umfasst.

3. Verfahren nach Anspruch 2, wobei die Bindungszusammensetzung einen Antikörper umfasst, der spezifisch an lösliches ST2 oder BNP bindet.

4. Verfahren nach Anspruch 1, wobei die CVD akutes Koronarsyndrom (ACS), Myokardinfarkt, Herzinsuffizienz, Angina pectoris, Herzyhypertrophie, Myokarditis, Perikarditis, Endokarditis oder Schlaganfall ist.

5. Verfahren nach Anspruch 1, wobei die PD COPD, Asthma, Lungenentzündung, Pneumothorax, Pleuraerguss, metastatische Krankheit, Lungenödem, Refluxösophagitis mit Aspiration, Lungenembolide oder eine restriktive Lungenerkrankung ist.

6. Verfahren nach Anspruch 1 oder 2, ferner umfassend Bestimmen eines Spiegels an einem oder mehreren anderen Biomarkern in der Probe.

7. Verfahren nach Anspruch 6, wobei der ein oder mehrere anderen Biomarker aus der nachfolgenden Gruppe gewählt sind: Creatinkinase MB (CK-MB), C-reaktives Protein (CRP), Kreatinin, und D-Dimere.

## Revendications

1. Procédé in vitro de réalisation d'un diagnostic différentiel entre maladie cardiovasculaire (MCV) et maladie pulmonaire (MP) chez un sujet présentant des douleurs thoraciques ou une dyspnée, le procédé comprenant :
la détermination d'un niveau de ST2 soluble (Interleukin 1 Receptor Like 1 (IL1RL1)) dans un échantillon prélevé sur le sujet ;
la détermination d'un niveau de peptide natriurétique de type B (BNP) dans l'échantillon ; et
la comparaison du niveau de ST2 soluble à un niveau de référence de ST2 soluble chez un sujet ne présentant ni MCV ni MP, ayant une forte probabilité de présenter une MP ou une MCV, ou présentant une MCV et/ou une MP ;
la comparaison du niveau de BNP de l'échantillon à un niveau de seuil bas de BNP de 100 pg/mL et à niveau de seuil haut de BNP de 500 pg/mL ; et
le diagnostic d'une MCV chez un sujet présentant des douleurs thoraciques ou une dyspnée, un niveau de ST2 soluble inférieur au niveau de référence de ST2 soluble et un niveau de BNP égal ou supérieur à 100 pg/mL ; ou
le diagnostic d'une MP chez un sujet présentant des douleurs thoraciques ou une dyspnée, un niveau de ST2 soluble égal ou supérieur au niveau de référence de ST2 soluble et un niveau de BNP égal ou inférieur à 500 pg/mL ; ou
le diagnostic d'une MCV chez un sujet présentant des douleurs thoraciques ou une dyspnée, un niveau de ST2 soluble égal ou supérieur au niveau de référence de ST2 soluble et un niveau de BNP supérieur à 500 pg/mL ;
l'échantillon consistant en du sang, du sérum ou du plasma.

2. Procédé selon la revendication 1, dans lequel la détermination du niveau de ST2 soluble ou du niveau de BNP dans l'échantillon comprend la mise en contact d'une composition de liaison avec l'échantillon, la composition de liaison se liant spécifiquement au ST2 soluble ou au BNP, et comprend la mesure ou la détermination de la liaison spécifique de la composition de liaison à l'échantillon.

3. Procédé selon la revendication 2, dans lequel la composition de liaison comprend an anticorps qui se lie spécifiquement au ST2 soluble ou au BNP.

4. Procédé selon la revendication 1, dans lequel la MCV est le syndrome coronarien aigu (SCA), l'infarctus du myocarde, l'insuffisance cardiaque, l'angor, l'hypertrophie cardiaque, la myocardite, la péricardite, l'endocardite ou l'accident vasculaire cérébral.

5. Procédé selon la revendication 1, dans lequel la MP est la broncho-pneumopathie chronique obstructive (BPCO), l'asthme, la pneumonie, le pneumothorax, l'effusion pleurale, une maladie métastatique, un oedème pulmonaire, le reflux gastro-oesophagien avec aspiration, l'embolie pulmonaire ou une maladie pulmonaire restrictive.

6. Procédé selon la revendication 1 ou 2, comprenant en outre la détermination d'un niveau, dans l'échantillon, d'un ou plusieurs autres biomarqueurs.

7. Procédé selon la revendication 6, dans lequel les un ou plusieurs autres biomarqueurs sont choisis dans le groupe constitué par : créatine kinase MB (CK-MB), protéine C-réactive (CRP), créatinine et D-dimères.
